# EUROPEAN PATENT APPLICATION

(11) **EP 1 811 031 A1**
(43) Date of publication of application: **25.07.2007**
(21) Application number: 06290126.9
(22) Date of filing: 18.01.2006
(51) Int. Cl.: C12N 15/10, C12N 15/62, C12P 21/00

(54) **Method for selecting a peptide or polypeptide which binds to a target molecule**

(71) Applicant: Millegen, 31670 Labege (FR)
(72) Inventor: Mondon, Philippe Lieudit Coteau de Fonbazi, 31450 Donneville (FR); Dubreuil, Olivier, 31190 Mauressac (FR); Carles, Marie-Julie, 31320 Castanet Tolosan (FR); Kharrat, Abdelhakim, 31450 Montgiscard (FR); Brune, Patrick, 31460 Segreville (FR)
(74) Representative: Nargolwalla, Cyra

(57) **Abstract**

A method for selecting a peptide or polypeptide which binds to a target is provided. The method is based on protein splicing and phage display.

## Description

### FIELD OF THE INVENTION

The present invention relates to phage display.

### BACKGROUND OF THE INVENTION

DNA recombination and genetic engineering techniques make it possible today to modify the structure of recombinant proteins or antibodies and evolve their functions. This is made possible by the contribution of modifications on the DNA sequence of a gene encoding for the aforementioned protein. These modifications corresponding to the creation of mutations can be carried out in a site directed or completely random way (for review see 1-3) and generate mutant libraries. The screening of these libraries allows selection of mutants presenting the required function.
One of the recent applications is the generation of recombinant antibody libraries. Libraries are generated starting from mRNA extracted from B cells, (from diverse lymphoid sources, taken among healthy subjects or patients suffering from various diseases) by PCR-based or similar cloning technology (4-6). These libraries can be optimised in term of diversity by the random incorporation of mutations on the heavy chains (VH) and light chains (VL) variable domains of immunoglobulins. Antibody libraries can be expressed as variable fragments (VH, VL, scFv or Fab). VH and VL variable domains of the antibody are responsible for the recognition and the binding to the antigen. Genetic engineering of this region helps the optimization of the immunologicals properties such as affinity, stability and specificity of an antibody for an antigen (7,8). The same approach is considered for the constant region (Fc region) of an antibody which carries binding epitope for many receptors, like effector cells of the immune system (for review see 9 and references therein).
Recombinant antibody libraries, naive or optimised by random mutagenesis, are of very significant size and very powerful selection tools are required in order to isolate the antibody of interest. Many of the selection platforms used today (bacterial, yeast and phage display) share four key steps: generation of genotypic diversity, coupling genotype to phenotype, application of selective pressure and amplification. Systems used today work on the basis of antibody expression (VH, VL, Fab or scFv fragment) on the surface of a cellular (bacterium, yeasts) or viral (phage) system. Phage display is the most popular system for antibody library screening (10) and relies on a strong binding of the antibody to the antigen which also makes it well suited to affinity maturation. However, this requires that the interaction between the antigen and the antibody is strong enough to be maintained until the end of the screening process and to allow the selection of the required antibody expressed on the phage cell-surface. In addition, when the antigen is a protein, the screening/selection process from an antibody library involves non specific or aspecific interactions which can generate many false positives. Thus, difficulty lies in the selection of mutants presenting a specific interaction with the antigen (or protein). In most of the current selection systems, identification of the specific interaction among the large non specific interactions requires many long and tedious stages.
In order to overcome these disadvantages, EP0614989 and some publications (11-13) describe a method for the selection of proteins which are involved in protein-ligand interaction. This method relates to the recovery of the infectious character of a phage displaying on its surface recombinant antibody fragment. The interaction between the antibody displayed on the phage surface and its ligand allows the restoration of the phage infecting ability. Indeed, this interaction occurs with the bringing together of two fragments of a viral coat protein (e.g. the minor coat protein pIII) which is essential to the phage infecting ability. However, this approach also suffers from several disadvantages. First, the infecting ability of the phage depends on strength of the interaction between the displayed protein and the ligand. Consequently, only strong or very strong affinity interactions will be able to keep together the two viral coat infectious protein fragments and restore the infecting ability of the phage. The outcome is a significant loss of interesting mutants in term of specificity. Mutants having a moderate to strong affinity, but being able to be the subject of an improvement during an additional mutagenesis-selection cycle will not be selected. In the case of a naive or randomly evolved antibody library, selection of an antibody with strong affinity to the antigen generally requires generation of different large size libraries and several mutagenesis-screening cycles to increase the success rate.
Furthermore, in the reaction medium containing the mutant library an important part of the ligand fused to the fragment of the viral coat protein remains free. During the selection step, this fusion molecule can bind to the host cells likely to be infected by the phages. Hence a competition with the phages with restored infecting ability takes place. There is then a phenomenon of exhaustion of the possibilities of connection to the host cell for the infectious phages. Thus, one observes a loss of a considerable proportion of the mutants with specific binding to the ligand.
Consequently it remains tedious to identify a peptide or a polypeptide which binds to a target from a random mutant library even using the more up to date phage display published methods. It is the object of the present invention to devise an improved method for selecting from a random protein variant library a peptide or polypeptide which binds to a target of interest.

### SUMMARY OF THE INVENTION

The present invention provides a versatile and sensitive method for selecting a peptide or polypeptide which binds to a target. The invention is based on protein trans-splicing and phage display.
Protein splicing is defined as the excision of an intervening sequence (the INTEIN) from a protein precursor and the concomitant ligation of the flanking protein fragments (the EXTEINS) to form a mature protein (extein) and the free intein. The intein plus the first C-extein residue (called the +1 amino acid) contain sufficient information to mediate splicing of the intein out of the protein precursor and ligation of the exteins to form a mature protein. Intein-mediated protein splicing results in a native peptide bond between the ligated exteins. It is now known that inteins incorporated into non-native precursors can also cause protein-splicing and excision of the inteins. In addition, an N-terminal intein fragment in a fusion protein and a C-terminal intein fragment in another fusion protein, when brought into contact with each other, can bring about trans-splicing between the two fusion proteins.
Thus, in accordance with the present invention, the protein splicing feature is used *in vitro* to transform a non-infectious virus into an infectious virus, thereby allowing the selection of a positive interaction of a peptide or polypeptide with a target. By using this method, extremely large libraries can be screened.
The present invention ensures a positive selection of the peptides or polypeptides of interest. The present invention allows the selection of peptides or polypeptides with a good specificity for a target and permits the improvement of their affinity for the target by successive mutagenesis rounds. The present invention is therefore well-suited to affinity maturation of antibodies in multiple rounds of mutation and selection.

### DETAILED DESCRIPTION OF THE INVENTION

The present invention provides a kit for selecting a peptide or polypeptide which binds to a target. The kit comprises:
a library of viruses each displaying on its surface a chimeric polypeptide of formula X-I₁-Z wherein X is a peptide or a polypeptide, I₁ is a first fragment of an intein and Z is a peptide or a protein which is present at the surface of each of said viruses, wherein each said virus comprises a nucleotide sequence encoding X and is not able to infect a host cell; and
an adapter molecule of formula A-I₂-C wherein A is a molecule which, when A is displayed on the surface of each said virus, renders the virus able to infect said host cell, I₂ is a second fragment of said intein and C is a target molecule, wherein X-I₁-Z and A-I₂-C are constructed in such a way that if X binds to C, A is covalently linked to Z upon trans-splicing through the first and the second fragments of said intein.

Typically the kit further comprises said host cell. Said host cell can be for example a prokaryote host cell and more particularly a bacterial host cell.
Typically any type of target molecule can be used. C can, for example, be selected from the group consisting of an antigen, an antibody, a nucleotide sequence, a receptor.

The three different components X, I₁, Z of the chimeric polypeptide of formula X-I₁-Z can be directly linked or linked via a spacer comprised of a peptide of 1 to 20 amino acids.

The three different components A, I₂, C of the adapter molecule can be directly linked or linked via a spacer comprised of a peptide of 1 to 20 amino acids. Alternatively the components can be linked together by using an appropriate chemical linking agent.

The virus to be used in the present invention can be any virus or viral vector. In a preferred embodiment the virus is a filamentous bacteriophage. For example said filamentous bacteriophage can be selected from the group consisting of Ff filamentous phage, lambda and T7. In particular, said filamentous bacteriophage is a Ff filamentous bacteriophage selected from the group consisting of fd, M13 and fl.

It falls within the ability of the skilled person to select Z, which is a protein or a peptide present at the surface of the virus. Z can be, depending on the virus used, a viral coat protein, a protein of the envelope of the virus, a protein of the capsid or a fragment thereof.

It falls within the ability of the skilled person to select molecule A which, when displayed on the surface of a virus renders the virus able to infect a host cell Techniques and molecules for altering the tropism of a virus are well known (see for example EP1191105 and WO2005040333). Molecule A, for example, can be selected from the group consisting of an antibody, a viral coat protein, a protein of the envelope of the virus, a protein of the capsid and fragment thereof.

In a preferred embodiment, Z is the C-terminal part of a surface protein of a virus which is required by said virus for the infection of a host cell and A is the N-terminal part of said surface protein.

For example if said virus is a filamentous bacteriophage, said surface protein can be selected from the group consisting of protein III (pIII) or protein VIII (pVIII). pIII of bacteriophage M13 comprises three domains of 68 (N1), 131 (N2) and 150 (CT) amino acids. pIII can be easily engineered in two pieces A and Z: the N-terminal part comprising domains N1 and N2: A and the C-terminal part comprising domain CT: Z. A phage only expressing at its surface the C-terminal part of pIII can not infect its traditional host cell. Infecting ability is restored when the N-terminal part of pill is linked the C-terminal part of pill.

In a preferred embodiment, X is an immunoglobulin, or a member of the immunoglobulin super-family, or any fragment thereof. In this context, the term immunoglobulin includes members of the classes IgA, IgD, IgE, IgG, and IgM. The term immunoglobulin super-family refers to all proteins which share structural characteristics with the immunoglobulins, including, for example, the T-cell receptor, or any of the molecules CD2, CD4, CD8 etc. Also included are fragments which can be generated from these molecules, such as Fv (a complex of the two variable regions of the molecule), single chain Fv (an Fv complex in which the component chains are joined by a linker molecule), Fab, F(ab')₂ or an immunoglobulin domain, such as the constant fragment (Fc), the variable heavy chain domain (VH) or the variable light chain domain VL.

It falls within the ability of the skilled person to select an intein and the two fragments thereof in order to construct X-I₁-Z and A-I₂-C in such a way that if X binds to C, A is covalently linked to Z upon trans-splicing through the first and the second fragments of said intein.
Typically the skilled person will use existing protocols to select the two fragments I₁ and I₂ and construct X-I₁-Z and A-I₂-C. Protein trans-splicing is a well known technique which has found a variety of applications including *in vitro* protein semisynthesis (21), segmental isotopic labeling (22), two and three hybrid strategies for monitoring protein activity in vivo (20, 23) and protein cyclization (24). Protein splicing permits the translation of an interaction event into a detectable signal through the reconstitution of a functional protein such as EGFP in *E coli* and yeast, and firefly luciferase in mammalian cells (see 23, 25-27 and EP1229330). In protein trans-splicing, a protein is split into two fragments and each half is fused to either the N-terminal or C-terminal fragments of an intein. Some inteins like the cis-splicing VMA intein from *Saccharomyces cerevisiae* have been engineered to be split in two fragments (N- and C-intein) to produce in vivo trans-spliced recombinant proteins (20). N-intein or C-intein alone is incapable of catalyzing protein splicing. However, when the N-intein and a C-intein, fused respectively to two interacting proteins, are in close proximity, they are capable of catalyzing protein trans-splicing.
Since the initial discovery of the VMA1 intein (14, 15), inteins have been identified in bacteria, archea and eukaryotic unicellular organisms (see The Intein Database and Registry http://www.neb.com/neb/inteins.html). Three Regions are found in each Intein: an N-terminal Splicing Region, a central Homing Endonuclease Region or a small central Linker Region, a C-terminal Splicing Region. Remarkably, inteins as small as 134 amino acids can splice out of precursor proteins. The discovery of mini-inteins and mutational analysis have indicated that the residues responsible for protein splicing are present in the N-terminal Splicing Region and the C-terminal Splicing Region (including the +1 amino acid in the C-extein). Several conserved motifs have been observed by comparing intein amino acid sequences. A nomenclature for these motifs has been defined (16): Blocks A, B, C, D, E, H, F, G. The N-terminal Splicing Region is about 100 amino acids and begins at the intein N-terminus and ends shortly after Block B. The intein C-terminal Splicing Region is usually less than 50 amino acids and includes Blocks F and G.
The N-terminal Splicing Region and the C-terminal Splicing Region form a single structural domain, which is conserved in all inteins studied to date.
Mini-inteins are usually about 130-200 amino acids. However, most inteins are greater than 300 amino acids, while the Pab RFC-2 intein is 608 amino acids. These big inteins have a larger linker region between intein Blocks B and F that includes intein Blocks C, D, E, and H homing endonuclease motifs.
The consensus sequence for blocks A, B, F and G is indicated below. Although no single residue is invariant, the Ser and Cys in Block A, the His in Block B, the His, Asn and Ser/Cys/Thr in Block G are the most conserved residues in the splicing motifs. Any member of an amino acid group may be present in the remaining positions, even when a specific predominant residue is indicated.
The upper case letters represent the standard single letter amino acid code for the most common amino acid at this position and lower case letters represent amino acid groups: x: any residue; x₁: C, S or T; h: hydrophobic residues: G,A,V,L,I,M; p: polar residues: S,C,T; a: acidic residues: D or E; r: aromatic residues: F,Y,W)
**Block A** (SEQ ID N°:1) : x₁hxxDpxhhhxxG (the first residue corresponds to the intein N-terminus)
**Block B** (SEQ ID N°:2): GxxhxhTxxHxhhh (usually 70-105 residues from N-terminus)
**Block F** (SEQ ID N°:3): rVYDLpV[1-3 residues]axx[H or E]NFh
**Block G** (SEQ ID N°:4): NGhhhHNp (p belongs to the downstream extein N-terminus)

In a preferred embodiment the intein is selected from the group consisting of DnaE, Ctr VMA, Mtu recA and Tac VMA.

In a preferred embodiment, Z is linked to the C-terminus of I₁ and I₁ comprises block F and block G and molecule A is linked to the N-terminus of I₂ and I₂ comprises block A and block B. Alternatively Z is linked to the N-terminus of I₁ and I₁ comprises block A and block B and molecule A is linked to the C-terminus of I₂ and I₂ comprises block F and block G.

In a further embodiment, the present invention relates to the virus comprising a nucleotide sequence encoding X and displaying on its surface a chimeric polypeptide of formula X-I₁-Z as described in the above mentioned kit.

In a further embodiment, the present invention relates to a library of viruses comprising a nucleotide sequence encoding X and displaying on its surface a chimeric polypeptide of formula X-I₁-Z as described in the above mentioned kit.

In a further embodiment, the present invention relates to the adapter molecule of formula A-I₂-C as described in the above mentioned kit.

In a further embodiment, the present invention relates to a vector comprising a nucleotide sequence encoding I₁-Z, wherein the vector is capable of being packaged into a virus and wherein the vector comprises a cloning site which enables the introduction of a nucleotide sequence encoding a peptide or polypeptide X in such a way that the chimeric polypeptide X-I₁-Z is displayed at the surface of said virus when said vector is packaged.
Typically the vector is a phagemid.

In a further embodiment, the present invention relates to a vector comprising a nucleotide sequence encoding X-I₁-Z, wherein the vector is capable of being packaged into a virus and wherein X-I₁-Z is displayed at the surface of said virus when said vector is packaged.

In a further embodiment, the present invention relates to a library of vectors comprising a nucleotide sequence encoding X-I₁-Z, wherein the vector is capable of being packaged into a virus and wherein X-I₁-Z is displayed at the surface of said virus when said vector is packaged.

In a further embodiment, the present invention relates to an expression vector comprising a nucleotide sequence encoding A-I₂, wherein said expression vector comprises a cloning site which enables the introduction of a nucleotide sequence encoding a target peptide or polypeptide C in such a way that a chimeric polypeptide of formula A-I₂-C can be expressed in a host cell. Typically this expression vector can be used for the production of the adapter molecule.

In a further embodiment, the present invention relates to a kit comprising:
a) a vector comprising a nucleotide sequence encoding I₁-Z, wherein the vector is capable of being packaged into a virus and wherein the vector comprises a cloning site which enables the introduction of a nucleotide sequence encoding a peptide or polypeptide X in such a way that the chimeric polypeptide X-I₁-Z is displayed at the surface of said virus when said vector is packaged; and
b) an expression vector comprising a nucleotide sequence encoding A-I₂, wherein said expression vector comprises a cloning site which enables the introduction of a nucleotide sequence encoding a target peptide or polypeptide C in such a way that a chimeric polypeptide of formula A-I₂-C can be expressed in a host cell.

In a further embodiment, the present invention relates to a method for producing a virus as described above comprising the step of genetically modifying a virus in such a way that when the virus is assembled the chimeric polypeptide of formula X-I₁-Z is displayed on the surface of the virus.
Typically the step of genetically modifying the virus can be performed by using the vector described above.

In a further embodiment, the present invention relates to a method for producing a library of viruses as described above comprising the steps of:
a) generating a library of vectors as described above, wherein each vector of the library comprises a variant nucleotide sequence encoding X;
b) genetically modifying viruses in such a way that when the viruses are assembled a chimeric polypeptide of formula X-I₁-Z is displayed on the surface of the viruses.

Typically the libraries result from the construction of nucleotide sequences repertories, nucleotide sequences characterised in that they are different by at least one change. The generation of the variant nucleotide sequences encoding X may be performed by site-directed mutagenesis, preferentially by random mutagenesis. Random mutagenesis can be performed by using a mutase, Pol beta for example (see W00238756).

In a further embodiment, the present invention relates to a method for selecting a peptide or polypeptide X which binds to a target C or a nucleotide sequence encoding X comprising the steps of:
a) combining the different components of the kit described above comprising a library of viruses and an adapter molecule, where said adapter molecule selectively interacts with viruses displaying a peptide or polypeptide X which binds to C, thereby conferring to these viruses the ability to infect the host cells;
b) replicating the viruses which are infective for the host cells by culturing the viruses in the presence of said host cells;
c) isolating from said host cells the viruses which replicate;
d) determining the nucleotide sequence encoding X from the viruses isolated in step c).

Optionally after step a) and before step b) the adapter molecules not having interacted with the viruses are removed.

In a further embodiment, the present invention relates to a method for producing a peptide or polypeptide X which binds to a target C comprising the steps of:
a) selecting the peptide or polypeptide X by performing the method described above; and
b) producing X.

In the following, the invention will be illustrated by means of the following non-limiting examples as well as the non-limiting figures.
Figures 1-2, 4-5 illustrate different constructs that allow expression of different fusion proteins used in the examples.
Figure 3 shows the type of ImmunoAssay used in the example to demonstrate the formation of a covalent link between two protein parts.

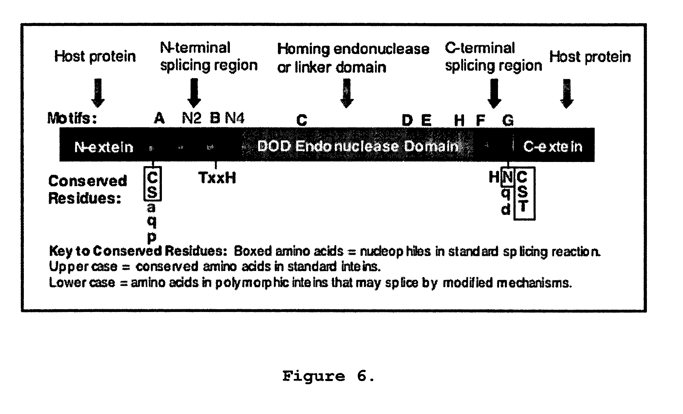

Figure 6 shows the different Intein motifs.

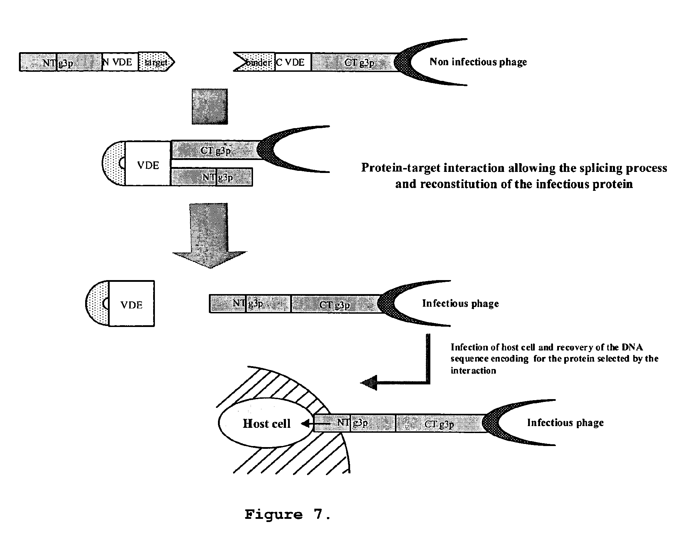

Figure 7 is a schematic diagram summarizing the present invention, in which Binder is X, CVDE is I₁, CTg3p is Z, NTg3p is A, NVDE is I₂ and target is C.

### Examples

In the following description, all molecular biology experiments are performed according to standard protocol (28).

### Example 1: Construction of the vectors for the protein-target interaction analysis

The intein used was a yeast VMA1-derived intein (VDE or PI-SceI) cloned in a pGEX vector: pGEX-VDE.
The protein III (abbreviated as pIII, gIIIp or g3p) of bacteriophage M13 consists of three domains of 68 (N1), 131 (N2) and 150 (CT) amino acids, connected by glycine-rich linker of 18 (G1) and 39 (G2) amino acids.

### a. Insertion of the intein in the gene III protein.

The gene of protein III (gene III) of bacteriophage M13 was PCR amplified and cloned in a pSK vector: pSK-GIII. Site directed mutagenesis was used to introduce SphI and AgeI restriction sites in the glycine-rich linker G2 of gene III using the primer pair: 5'-GGCGGTTCTGAGGGTGGCGCATGCGAGGGAGGCGGCGGTTCCGG-3' (SEQ ID N°:5) and 5'-CCGGAACCGCCTCCCTCGCATGCGCCCACCCTCAGAACCGCC-3' (SEQ ID N°:6) and the primer pair 5'-GAGGGAGGCGGTACCGGTGGTGGCTCTGG-3' (SEQ ID N°:7) and 5'-CCAGAGCCACCACCGGTACCGCCTCCCTC-3' (SEQ ID N°:8), respectively.

The N-terminal domain of the VDE (N-VDE: amino acids 1 to 187) were PCR amplified from pGEX-VDE using the primer pair: 5'-GCATGCTTTGCCAAGGGTACCAATG-3' (SEQ ID N°:9) and 5'-CTCGAGTGTGCCGTTGCCGTTGTTTCTGTCATTCTCATAAAGAATTGGAGCG-3' (SEQ ID N°: 10) which allows to add *Sph*I and *Xho*I restriction sites at the two extremities of the N-VDE.

The C-terminal domain (C-VDE: amino acids 388 to 455) of the VDE was amplified using the primer pair CTCGAGAGAAACAACGGCAACGGGAACGGCACAGGAGATGTTTTGCTTAACGT (SEQ ID N°: 11) and ACCGGTACCGCCTCCCTCGCAATTGTGGACGACAACCTGGGATCC (SEQ ID N°:12) which allows to add *Xho*I and *Age*I restriction sites at the two extremities of the C-VDE and a linker at the N-terminal part of the C-VDE.

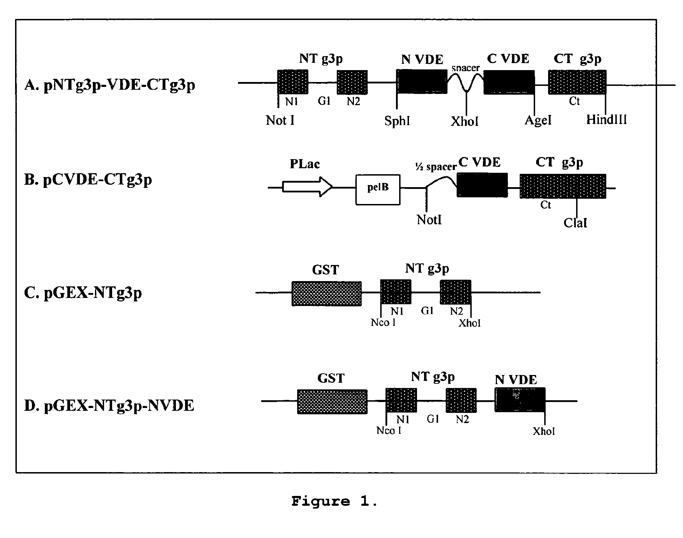

The N-terminal and the C- terminal domains of the VDE amplified from the plasmid pGEX-NVDE were then cloned into the *Sph*I - *Age*I restriction sites of the gene III to obtain the vector pNTg3p-VDE-CTg3p (Figure 1A) with the fusion protein: NTg3p (N1-N2 of pIII)-NVDE-linker-C-VDE-CTgIIIp).

### b. Construction of the phagemid with C-extein of the VDE in fusion with CT of pIII.

The C-VDE and CT of pIII (CTg3p) fusion protein was PCR amplified from the vector pNg3p-VDE-CTg3p using the primer pair 5'-ATAAGAATGCGGCCGCATAGAGAAACAACGGCAACGGGAACGG-3' (SEQ ID N°:13) and TAATACGACTCACTATAGGG (SEQ ID N°:14), which allows to replace *Xho*I by *Not*I at the N-terminal and cloned between the *Not*I and *Cla*I restriction sites of the vector pSK-GIII in fusion with a signal sequence pelB and under the control of a Lac promoter to generate the phagemid pCVDE-CTg3p (Figure 1B).

### c. Construction of the vector to express the target in fusion with N1-N2 domain (NTg3p) of gIIIp the half VDE (N-VDE).

The Nl-N2 domain of the gene III was PCR amplified from the vector pNP3-VDE-CP3 using the primer pair 5'-CCATGGCTGAAACTGTTGAAAGTTGTTTAGC-3' (SEQ ID N°:15) and 5'-CTCGAGGCATGCGCCACCCTCAGAACC-3' (SEQ ID N°:16) which allows to add *Nco*I in 5' and *Xho*I in 3' and cloned in the *Nco*I and *Xho*I restriction sites of the pGEX vector to generate the control plasmid pGEX-NTg3p (Figure 1C).

The N1-N2-NVDE fusion protein gene was PCR amplified from the vector pNTg3p-VDE-CTg3p using the primer pair 5'-CCATGGCTGAAACTGTTGAAAGTTGTTTAGC-3' (SEQ ID N°:17) and 5'-CTCGAGTGTGCCGTTGCCGTTGTTTCTGTCATTCTCATAAAGAATTGGAGCG-3' (SEQ ID N°:18) in order to insert *Nco*I in 5' and cloned in the *Nco*I and *Xho*I restriction sites of the pGEX vector providing the plasmid pGEX-NTg3p-NVDE (Figure 1D).

The N1-N2-NVDE fusion protein gene was PCR amplified from the vector pGEX-NTg3p-NVDE using the primer pair 5'-TATAGTATGAGCTCGCCATGGCTGAAACTGTTGAAAGTTG-3' (SEQ ID N°:19) and 5'-TATATAGAATTCTCACTTCTTCTCGAGTGTGCCGTTCCCGTT-3' (SEQ ID N°:20) in order to insert *Sac*I in 5'end and *Eco*RI, and 2 codons for lysine in 3'end and cloned in the *Sac*I and *Eco*RI restriction sites of the expression pMG20 vector (MilleGen) providing the plasmid **pMG20-NTg3p-NVDE_K.**

### Example 2: Reconstitution of the two portions of the gene III protein via protein-target interaction of a phage displaying an anti N-VEGF antibody and the N portion of the VEGF.

### a. Phage fusion antibody anti-NVEGF

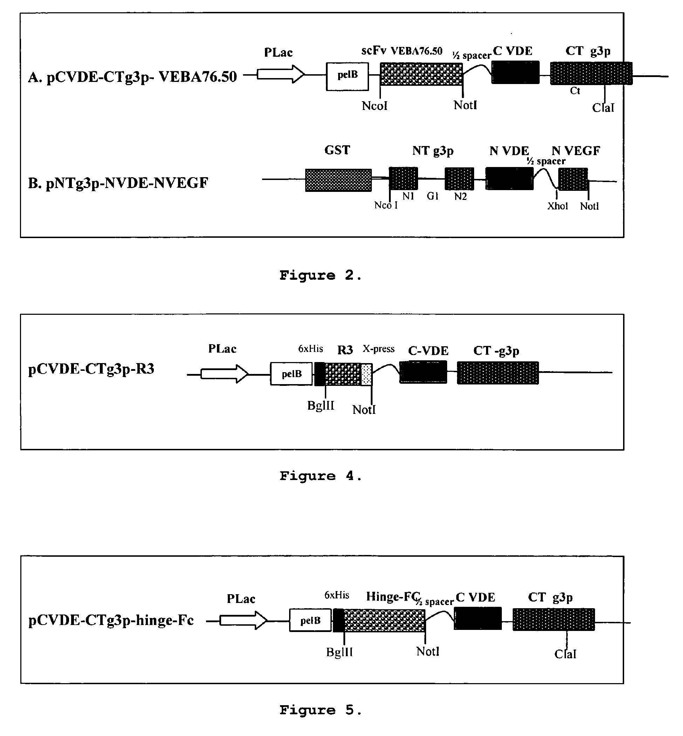

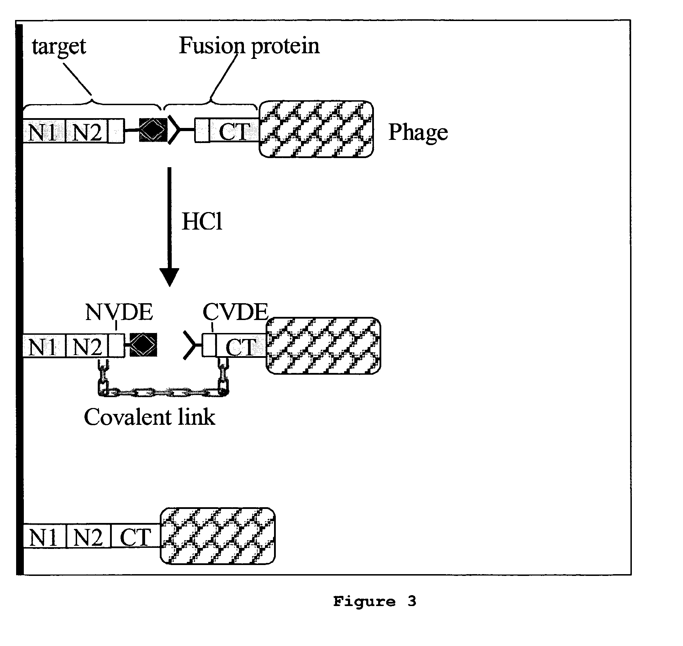

The retrotranscript of the VH and VL genes of the hybridoma VEBA76.50 were PCR amplified and a single chain antibody Fv fragment (scFv) having the structure VH-VL was cloned into the vector pCR4-topoTA (Invitrogen). The VEBA76.50 scFV was digested with NcoI and NotI and cloned into the phagemid pCextein-CTg3p digested with the same enzymes giving the phagemid pCVDE-CTg3p-VEBA76.50 (Figure 2A).

This phagemid encodes the VEBA76.50 scFv as an N-terminal fusion of C-terminal domain of the intein (CVDE) and the C-terminal domain of the pIII. Phage particles displaying the scFv on their surfaces were produced in the E. *coli* XL1blue harbouring the plasmid pCVDE-CTg3p-VEBA76.50 and co-infected with the hyperphage M13KO7ΔpIII (Progen). The phages were then prepared according to standard methods (28).

Competitive ELISA was used to characterise the phage particles displaying on their surface the fusion protein scFv-CVDE-CTg3p. The phage particles were added to each well of microtiter plates previously coated with the fusion protein GST-NVEGF and incubated 2h at 37°C in the presence of decreasing concentration of soluble GST-NVEGF used as competitor. After three washes, phages that bound to the wells were detected with a peroxidase conjugate anti-M13 antibody and TMB (Sigma). The inhibition curves obtained permit to determinate the relative affinity of the VEBA76.50-CVDE-CTg3p-Phages for the N terminal part of the VEGF (NVEGF). In this case the deduced relative affinity for the NVEGF was in the nanomolar range (20nM).

### b. Construction of the target complex

The N portion of the VEGF was PCR amplified from the vector pGEX_NVEGF using the primer pair 5'-CTCGAGCGGCGGCGGACAGTGGACGCG-3' (SEQ ID N°:21) and 5'-GCGGCCGCTTACCGGGCCAGGGCCTGGGGAGC-3' (SEQ ID N°:22) was cloned into the vector pCR4-Topo. The NVEGF was digested with *Xho*I and *Not*I and cloned into the pGEX-NTg3p-NVDE digested with the same enzymes, giving the vector pNTg3p-NVDE-NVEGF (Figure 2B).

The target fusion complex NTg3p-NVDE-NVEGF produced in *E coli* strain BL21(DE3) are purified using a glutatione chromatography according to standard methods (28).
A competitive ELISA was done to evaluate the binding of the phage particules to the target fusion complex. The protocole was the same as described previously but in this case the wells were coated with the target complex fusion (GST-NTg3p-NVDE-NVEGF). As a result, the phage displaying the antibody fusion complex binds specifically the N terminal part of the VEGF of the target fusion complex.

### c. Formation of a covalent link via trans-splicing following protein-target interaction

During trans-splicing process, the intein was reconstituted and a covalent link occurred with the flanking sequence named extein. The formation of a covalent link between two protein parts can be demonstrated by a particular type of ImmunoAssay (Figure 3). This assay requires different steps as described as follow: i) the fusion target complex NTg3p-NVDE-NVEGF was coated to a 96-wells microtiter plate, ii) different dilutions in the splicing buffer of the phage fusion anti-body displaying VEBA76.50-CVDE-CTg3p were added and incubated 5h (or overnight) at 24°C, iii)after three washes, the non covalently link scFv fusion phages were released by the addition of a dissociating agent (HCl) and were removed by a subsequently step of washing, iv) despite the treatment with dissociating agent, the covalent bound scFv fusion phages due to trans-splicing event with the target fusion complex immobilised on the microtiter plate were not released and were revealed with an anti-fd phage antibody peroxidase conjugate as described before. Phages displaying the same fusion protein without the N terminal part of the VDE were used as control.

### Example 3: Reconstitution of the two portions of the gene III protein via protein-target interaction of a phage displaying a peptide anti-RhoB (R3) and the RhoB protein.

### a. Phage fusion complex

A peptide anti-RhoB was isolated from a highly diversify antibody library (MutalBank-Millegen) through a screening against RhoB protein. The peptide R3 (25 aa) has a specific affinity against the protein RhoB. The peptide R3 was PCR amplified with the primers pair 5'-GCAGCCCCATAAACACACAGTATGT-3' (SEQ ID N°:23) and 5'-ATATATATGCGGCCGCCTTATCGTCATCGTCGTACAGATCTGAACCGCCTCCACCAC TCCGCTCGAGGAGATGGATTGTAGCGCTTATCATC-3' (SEQ ID N°:24) in order to insert NotII, a GS linker, a TAG (Xpress) in 3' and cloned in the BglII and NotI restriction site of the phagemid pCextein-CTg3p-hinge-Fc to obtain pCVDE-CTg3p-R3 (Figure 4). Phage particles were produced in the *E*. *coli* XL1blue harbouring the pCVDE-CTg3p-R3 and co-infected with the hyperphage M13KO7ΔpIII (Progen) . The phages were then prepared according to standard methods. The phages displaying on their surface the fusion protein R3-CVDE-CTg3p that specifically recognised RhoB protein was checked by ELISA.

### b. Construction of the target complex

RhoB gene was PCR amplified from the vector pIRES-puro-HA-RhoB (29) using the primer pair 5'-TATAGGTCGACATGGCTTACCCATACGATGTTCCAGA-3' (SEQ ID N°:25) and 5'- TATATATCTAGATAGCACCTTGCAGCAGTTGATGCA-3' (SEQ ID N°:26) and was cloned into the vector pCR4-topoTA (Invitrogen). The plasmid pCR4-topoTA-RhoB was digested with SalI and EcoRI and the insert was cloned in the XhoI and EcoRI restriction sites of the plasmid pMG20-NTg3p-Nextein_K to obtain pMG20-Nl-N2-Nextein_RhoB. The fusion protein NTg3p-NVDE_RhoB was expressed in *E. coli* strain BL21DE3 and purified by Ni-NTA chromatography according to standard methods (28).

### c. Restoration of phage infecting ability through the reconstitution of the gene III

R3-CVDE-CTg3p fusion phages were incubated with the target complex NTg3p-NVDE_RhoB in the splicing buffer 18h at 24°C. This mixture was added to an excess of *E*. *coli* XL1blue cells and after incubation at 37°C, aliquots were plated on 2YT-agar containing 100µg/ml of ampicillin, 0.5% glucose. Phages recovering infecting ability were counted as colony forming units after overnight incubation at 37°C.

### Example 4: Reconstitution of the two portions of the gene III protein via protein-target interaction of a phage displaying a hinge-Fc fragment and the protein A from Staphylococcus aureus.

### a. Phage fusion complex

The fragment hinge-Fc (aa: 216-447) of a human IgG1 has been amplified from the clone pBHuCγ1 (30) with the primer pair 5'-ATATATATAGCCATGGCGGGGGGTTCTCACCACCATCACCACCACGGGAGATCTGGA TCCGAGCCCAAATCTTGTGA-3' (SEQ ID N°:27) and 5'-GCTAGTCAGTGCGGCCGCGAATTCTTTACCCGGAGACAGGGAGAG-3' (SEQ ID N°:28) in order to insert NcoI, a strech of six histidines and BglII in 5' and NotI in 3'. The PCR product was digested and cloned in the NcoI and NotI restriction sites of the phagemid pCVDE-CTg3p vector providing the phagemid pCVDE-CTg3p-hinge-Fc (Figure 5). Phage particles displaying the fusion complex hinge-Fc CVDE-CTg3p on their surface were generated in the *E. coli* XL1blue harbouring the pCextein-CTg3p-hinge-Fc phagemid through a co-infection with the hyperphage M13KO7ΔpIII (Progen). The phages were then prepared according to standard methods.

### a. Target complex

Production of N1-N2-NVDE_K and coupling to protein A (spA).
The fusion protein NTg3p-Nextein_K was expressed using the plasmid pMG20-Nl-N2-NVDE_K in E. *coli* strain BL21DE3 and purified by Ni-NTA chromatography according to standard methods. NTg3p-Nextein_K was coupled with Protein A in molar ratio 1/1 on free primary amine (Lysin lateral chain) by the water soluble homo bifunctional glutaraldehyde.
Coupling product was subjected to an IMAC purification procedure on a NiNTA Agarose resin (Qiagen) followed by a size exclusion gel chromatography (Amersham). The resulting complex was analysed by SDS PAGE and western blot.

### b. Restoration of Phage infecting ability

The hinge-Fc-CVDE-CTg3p fusion phage were incubated with the target complex N1-N2-NVDE_hinge_Fc in the splicing buffer 18h at 24°C. This mixture was added to an excess of *E*. *coli* XL1blue cells and after incubation at 37°C, aliquots were plated on 2YT-agar containing 100µg/ml of ampicillin, 0.5% glucose. Phages recovering infecting ability were counted as colony forming units after incubation overnight at 37°C.

### Example 5. Reconstitution of the two portions of pIII via protein-target interaction of a phage displaying a VH anti-Klip1 antibody and the Klip1 protein.

### a. Phage fusion complex

A domain of variable heavy chain was isolated from a highly diversify antibody library (MutalBank-Millegen) through a screening against Klip-1 extracellular fragment. The VH-4K has a specific affinity against the Klip-1 extracellular fragment. The antibody fragment VH-4K was PCR amplified with the primers pair 5'-GCAGCCCCATAAACACACAGTATGT-3' (SEQ ID N°:29) and 5'-ATATATATATGCGGCCGCGAATTCGAAGATCCGCCGCCAC-3' (SEQ ID N°:30) in order to insert NotI in 3' and cloned in the BglII and NotI restriction site of the phagemid pCVDE-CTg3p-hinge-Fc to replace the hinge-Fc with VH-4k to obtain pCVDE-CTg3p-VH-4k. Phage particles displaying the fusion complex VH-4k-CVDE-CTg3p on their surface were produced in the *E*. *coli* XL1blue harbouring pCVDE-CTg3p-VH-4k and co-infected with the hyperphage M13KO7ΔpIII (Progen). The phages were then prepared according to standard methods and the affinity to Klip1 protein was checked by ELISA.

### b. Target complex

Klip-1 extracellular fragment was PCR amplified from the vector pQE-31 (31) using the primer pair 5'-TATATACTCGAGGAAGAAAACATCCAGGGCGGAG-3' (SEQ ID N°:31) and 5'-TATATATCTAGAAGGTCCATAGAGTTCACCTG-3' (SEQ ID N°:32) was cloned into the vector pCR4-topoTA (Invitrogen). Klip-1 was removed from plasmid pCR4-topoTA-Klip-1 and cloned in the XhoI and EcoRI restriction sites of the plasmid pMG20-NTg3p-NVDE_K to obtain pMG20-NTg3p-NVDE_Klip1. The fusion protein NTg3p-NVDE_Klip1 was expressed in *E*. *coli* strain BL21DE3 and purified by Ni-NTA chromatography according to standard methods (28).

### c. Restoration of phage infecting ability

VH-4k-CVDE-CTg3p fusion phage were incubated with the target complex NTg3p-NVDE_Klip1 in the splicing buffer 18h at 24°C. This mixture was added to an excess of *E*. *coli* XL1blue cells and after incubation at 37°C, aliquots were plated on 2YT-agar containing 100µg/ml of ampicillin, 0.5% glucose. Phages recovering infecting ability were counted as colony forming units after incubation overnight at 37°C.

### References:

Throughout this application, various references describe the state of the art to which this invention pertains. The disclosures of these references are hereby incorporated by reference into the present disclosure.
1. Ling M and Robinson BH, (1997), Approaches to mutagenesis: an overview. Anal. Biochem., 254, 157-178.
2. Scandalis A, Encell LP and Loeb LA, (1997), Creating novel enzymes by applied molecular evolution. Chem. Biol., 4, 889-898.
3. Minshull J and Stemmer WPC, (1999), Protein evolution by molecular breeding. Curr. Opin. Chem. Biol., 3, 284-290.
4. Orlandi R, Gussow DH, Jones PT and Winter G, (1989), Cloning immunoglobulin variable domains for expression by the polymerase chain reaction. 86, 3833-3837.
5. Huse, WD et al., (1989), Generation of a large combinatorial library of the immunoglobulin repertoire in phage lambda. Science, 246, 1275-1281.
6. Schoonbroodt, S et al., (2005), Oligonucleotide-assisted cleavage and ligation: a novel directional DNA cloning technology to capture cDNAs. Application in the construction of a immune antibody phage display library. Nucleic Acids Res., 33, e81.
7. Gram H, Marconi, LA, Barbara CF, Collet, TA, Lerner RA and Kang AS,(1992), in vitro selection and affinity maturation of antibodies from a naive combinatorial immunoglobulin library. Proc. Natl. Acad. Sci., 89, 3576-3580.
8. Marks JD, Griffiths AD, Malmqvist M, Clackson TP, Bye JM and Winter G, (1992), By passing immunization: building high affinity human antibodies by chain shuffling. Biotechnology, 10, 779-783.
9. Carter P, (2001),Improving the efficacy of the antibody-based cancer therapies. Nature, 1, 118-129.
10. Smith GP, (1985), Filamentous fusion phage: novel expression vectors that display on the virion surface. Science, 228, 1315.
11. Krebber C, Moroney S, Plückthum A and Schneider C, (1994), A method for in vitro selection of ligand-binding proteins.
12. Krebber C, Spada S, Desplancq D and Plückthun A, (1995), Co-selection of cognate antibody-antigen pairs by selectivity-infective phages. FEBS letters, 377, 227-231.
13. Krebber C, Spada S, Desplancq D, Krebber A, Ge L, and Plückthum A, (1997), Selectively infective phage (SIP) : A mechanistic dissection of a novel in vivo selection for protein-ligand interactions. J. Mol. Biol., 268, 607-618.
14. Hitara et al (1990) Molecular structure of a gene, VMA1, encoding the catalytic subunit of H+-translocating adenosine triphosphatase from vacuolar membranes of Saccharomyces cerevisiae. J Biol chem, 265,6726-6733.
15. Kane et al (1990) Protein splicing converts the yeast TFP1 gene product to the 69 kD subunit of the vacuolar H+-adenosine triphosphate. Science, 250,651-657.
16. Pietrokovski S (1994) Conserved sequence features of inteins (protein introns) and their use in identifying new inteins and related proteins. Protein Sci., 3:2340-2350.
17. Chong S and Xu MQ (1997) Protein splicing of the Saccharomyces cerevisiae VMA Intein without the endonuclease motifs. J biol Chem 272, 15587-15590.
18. Zhang A, Gonzalez SM, Cantor EJ and Chong S (2001) Construction of a mini-intein fusion system to allow both direct monitoring of soluble protein expression and rapid purification of target proteins. Gene 275, 241-252.
19. Wu H, Hu Z and Lui XQ. (1998) Protein trans-splicing by a split intein encoded in a split DnaE gene of Synechocystis sp. PCC6803.Proc. Natl. Acad. Sci., 95, 9226-9231.
20. Ozawa T et al. (2000) A fluorescent indicator for detecting protein-protein interaction in vivo based on protein splicing. Anal Chem, 72, 5151-5157.
21. Lew BM, Mills KV and Paulus H (1998) Protein splicing in vitro with semisynthetic two-component minimal intein.. J Biol Chem 273, 15887-15890.
22. Otomo T, Teruya K, Uegaki K, Yamazaki T and Kyogoku Y (1999) Improved segmental isotope labeling of proteins and application to a larger protein. J Biomol NMR. 14, 105-14.
23. Mootz HD, Blum ES, Tyszkiewicz AB and Muir TW (2003) Conditional protein splicing: A new tool to control protein structure and function in vitro and in vivo. J Am Chem Soc, 125,10561-10569.
24. Evans TC, Benner J and Xu MQ (1999) The cyclisation and polymerisation of bacterially expressed proteins using modified self-splicing inteins. J Biol Chem 274, 18359-18363.
25. Ozawa T and Umezawa Y. (2001) Detection of protein-protein interactions in vivo based on protein splicing. Curr Opin Chem Biol, 5, 578-583.
26. Shi J and Muir TW (2005) Development of a tandem protein trans-splicing system based on native and engineered split inteins. J Am Chem Soc, 127,6198-6206.
27. Xu MQ and Evans TC (2005) Recent advances in protein splicing: manipulating proteins in vitro and in vivo. Curr Opin Chem Biol, 16, 440-446
28. Sambrook J, Fritsch EF and Maniatis T (eds) Molecular cloning, A laboratory Manual 2nd Ed, Cold Spring Harbor Laboratory Press.
29. Baron R, Fourcade E, Lajoie-Mazenc I, Allal C, Couderc B, Barbaras R, Favre G, Faye JC, Pradines A (2000) RhoB prenylation is driven by the three carboxyl-terminal amino acids of the protein: Evidenced in vivo by an anti-farnesyl cysteine antibody. Proc Natl Acad Sci U S A. 97,11626-11631.
30. Poul MA, Cerutti M, Chaabihi H, Devauchelle G, Kaczorek M, Lefranc MP (1995). Design of cassette baculovirus vectors for the production of therapeutic antibodies in insect cells. Immunotechnology. 1 : 189-196.
31. Prost S, LeDiscorde M, Haddad R, Gluckman JC, Canque B and Kirszenbaum M. (2002) Characterization of a Novel Hematopoietic Marker Expressed from Early Embryonic Hematopoietic Stem Cells to Adult Mature Lineages. Blood Cells Mol Dis., 29, 236-48.

## Claims

1. A kit comprising:
a library of viruses each displaying on its surface a chimeric polypeptide of formula X-I₁-Z wherein X is a peptide or a polypeptide, I₁ is a first fragment of an intein and Z is a peptide or a protein which is present at the surface of each of said viruses, wherein each said virus comprises a nucleotide sequence encoding X and is not able to infect a host cell; and
an adapter molecule of formula A-I₂-C wherein A is a molecule which, when A is displayed on the surface of each said virus, renders the virus able to infect said host cell, I₂ is a second fragment of said intein and C is a target molecule, wherein X-I₁-Z and A-I₂-C are constructed in such a way that if X binds to C, A is covalently linked to Z upon trans-splicing through the first and the second fragments of said intein.

2. The kit of claim 1 further comprising said host cell, preferably a bacterial host cell.

3. The kit according to claim 1 or 2 wherein C is selected from the group consisting of an antigen, an antibody, a nucleotide sequence, a receptor.

4. The kit according to any of claims 1 to 3, wherein said virus is a phage, preferably a filamentous bacteriophage.

5. The kit according to any of claims 1 to 4 wherein Z is selected from the group consisting of a viral coat protein, a protein of the envelope of the virus, a protein of the capsid and fragment thereof.

6. The kit according to any of claims 1 to 5 wherein A is selected from the group consisting of an antibody, a viral coat protein and fragment thereof.

7. The kit according to any of claims 1 to 6, wherein Z is the C-terminal part of a surface protein of a virus which is required by said virus for the infection of a host cell and A is the N-terminal part of said surface protein.

8. The kit according to claim 7 wherein said virus is a filamentous bacteriophage and said surface protein is selected from the group consisting of pIII and pVIII.

9. The kit according to any of claims 1 to 8, wherein X is selected from the group consisting of an immunoglobulin, a member of the immunoglobulin super-family, and fragment thereof.

10. The kit according to any of claims 1 to 9 wherein the intein is selected from the group consisting of DnaE, Ctr VMA, Mtu recA and Tac VMA.

11. The kit, according to any of claims 1 to 10, wherein Z is linked to the C-terminus of I₁ and I₁ comprises block F (SEQ ID: 3) and block G (SEQ ID: 4) and molecule A is linked to the N-terminus of I₂ and I₂ comprises block A (SEQ ID: 1) and block B (SEQ ID: 2).

12. The kit, according to any of claims 1 to 10, wherein Z is linked to the N-terminus of I₁ and I₁ comprises block A and block B and molecule A is linked to the C-terminus of I₂ and I₂ comprises block F and block G.

13. A virus as described in any of claims 1 to 12 comprising a nucleotide sequence encoding X and displaying on its surface a chimeric polypeptide of formula X-I₁-Z

14. A library of viruses as described in any of claims 1 to 12.

15. An adapter molecule of formula A-I₂-C as described in any of claims 1 to 12.

16. A vector, preferably a phagemid, comprising a nucleotide sequence encoding the chimeric polypeptide I₁-Z, wherein the vector is capable of being packaged into a virus and wherein the vector comprises a cloning site which enables the introduction of a nucleotide sequence encoding a peptide or polypeptide X in such a way that the chimeric polypeptide X-I₁-Z as described in any of claims 1 to 12 is displayed at the surface of said virus when said vector is packaged.

17. A vector, preferably a phagemid, comprising a nucleotide sequence encoding X-I₁-Z as described in any of claims 1 to 12, wherein the vector is capable of being packaged into a virus and wherein X-I₁-Z is displayed at the surface of said virus when said vector is packaged.

18. A library of vectors as described in claim 17.

19. An expression vector comprising a nucleotide sequence encoding A-I₂, wherein said expression vector comprises a cloning site which enables the introduction of a nucleotide sequence encoding a target peptide or polypeptide C in such a way that a chimeric polypeptide of formula A-I₂-C as described in any of claims 1 to 12 can be expressed in a host cell.

20. A kit comprising:
a) a vector according to claim 16; and
b) an expression vector according to claim 19.

21. A method for producing a virus according to claim 13 comprising the step of genetically modifying a virus in such a way that when the virus is assembled the chimeric polypeptide of formula X-I₁-Z is displayed on the surface of the virus.

22. A method for producing a library of viruses according to claim 14 comprising the steps of:
a) generating a library of vectors according to claim 18, wherein each vector of the library comprises a variant nucleotide sequence encoding X;
b) genetically modifying viruses in such a way that when the viruses are assembled a chimeric polypeptide of formula X-I₁-Z is displayed on the surface of the viruses.

23. The method for making the library of viruses of claim 22 wherein the variant nucleotide sequences encoding X are generated by random mutagenesis.

24. A method for selecting a peptide or polypeptide X which binds to a target C or a nucleotide sequence encoding X comprising the steps of:
a) combining the different components of the kit according to any of claims 1 to 13, where said adapter molecule selectively interacts with viruses displaying a peptide or polypeptide X which binds to C, thereby conferring to these viruses the ability to infect the host cells;
b) replicating the viruses which are infective for the host cells by culturing the viruses in the presence of said host cells;
c) isolating from said host cells the viruses which replicate;
d) determining the nucleotide sequence encoding X from the viruses isolated in step c).

25. The method of claim 24 wherein after step a) and before step b) the adapter molecules not having interacted with the viruses are removed.

26. A method for producing a peptide or polypeptide X which binds to a target C comprising the steps of:
a) selecting the peptide or polypeptide X by performing the method of claim 24; and
b) producing X.


SEQUENCE LISTING

<110>  MilleGen

<120>  Method for selecting a peptide or polypeptide which binds to a
       target

<130>  BEP050541

<160>  32

<170>  PatentIn version 3.3

<210>  1
<211>  13
<212>  PRT
<213>  Artificial

<220>
<223>  block A


<220>
<221>  MISC_FEATURE
<222>  (1)..(1)
<223>  Xaa can be C, S or T

<220>
<221>  MISC_FEATURE
<222>  (2)..(2)
<223>  Xaa can be G, A, V, L, I, M

<220>
<221>  misc_feature
<222>  (3)..(4)
<223>  Xaa can be any naturally occurring amino acid

<220>
<221>  MISC_FEATURE
<222>  (6)..(6)
<223>  Xaa can be S, C, T

<220>
<221>  misc_feature
<222>  (7)..(7)
<223>  Xaa can be any naturally occurring amino acid

<220>
<221>  MISC_FEATURE
<222>  (8)..(10)
<223>  Xaa can be G, A, V, L, I, M

<220>
<221>  misc_feature
<222>  (11)..(12)
<223>  Xaa can be any naturally occurring amino acid

<400>  1

Xaa Xaa Xaa Xaa Asp Xaa Xaa Xaa Xaa Xaa Xaa Xaa Gly
1               5                   10


<210>  2
<211>  14
<212>  PRT
<213>  Artificial

<220>
<223>  block B


<220>
<221>  misc_feature
<222>  (2)..(3)
<223>  Xaa can be any naturally occurring amino acid

<220>
<221>  MISC_FEATURE
<222>  (4)..(4)
<223>  Xaa can be G, A, V, L, I, M

<220>
<221>  misc_feature
<222>  (5)..(5)
<223>  Xaa can be any naturally occurring amino acid

<220>
<221>  MISC_FEATURE
<222>  (6)..(6)
<223>  Xaa can be G, A, V, L, I, M

<220>
<221>  misc_feature
<222>  (8)..(9)
<223>  Xaa can be any naturally occurring amino acid

<220>
<221>  misc_feature
<222>  (11)..(11)
<223>  Xaa can be any naturally occurring amino acid

<220>
<221>  MISC_FEATURE
<222>  (12)..(14)
<223>  Xaa can be G, A, V, L, I, M

<220>
<221>  MISC_FEATURE
<222>  (12)..(14)
<223>  Xaa can be G, A, V, L, I, M

<400>  2

Gly Xaa Xaa Xaa Xaa Xaa Thr Xaa Xaa His Xaa Xaa Xaa Xaa
1               5                   10


<210>  3
<211>  15
<212>  PRT
<213>  Artificial

<220>
<223>  block F


<220>
<221>  MISC_FEATURE
<222>  (1)..(1)
<223>  Xaa can be F, Y, W

<220>
<221>  MISC_FEATURE
<222>  (6)..(6)
<223>  Xaa can be S, C, T

<220>
<221>  misc_feature
<222>  (8)..(8)
<223>  Xaa can be any naturally occurring amino acid

<220>
<221>  MISC_FEATURE
<222>  (9)..(9)
<223>  Xaa can be D, E

<220>
<221>  misc_feature
<222>  (10)..(11)
<223>  Xaa can be any naturally occurring amino acid

<220>
<221>  MISC_FEATURE
<222>  (12)..(12)
<223>  Xaa can be H, E

<220>
<221>  MISC_FEATURE
<222>  (15)..(15)
<223>  Xaa can be G, A, V, L, I, M

<400>  3

Xaa Val Tyr Asp Leu Xaa Val Xaa Xaa Xaa Xaa Xaa Asn Phe Xaa
1               5                   10                  15


<210>  4
<211>  8
<212>  PRT
<213>  Artificial

<220>
<223>  block G


<220>
<221>  MISC_FEATURE
<222>  (3)..(5)
<223>  Xaa can be G, A, V, L, I, M

<220>
<221>  MISC_FEATURE
<222>  (8)..(8)
<223>  Xaa can be S, C, T

<400>  4

Asn Gly Xaa Xaa Xaa His Asn Xaa
1               5


<210>  5
<211>  44
<212>  DNA
<213>  Artificial

<220>
<223>  primer

<400>  5
ggcggttctg agggtggcgc atgcgaggga ggcggcggtt ccgg                        44

<210>  6
<211>  42
<212>  DNA
<213>  Artificial

<220>
<223>  primer

<400>  6
ccggaaccgc ctccctcgca tgcgcccacc ctcagaaccg cc                    42


<210>  7
<211>  29
<212>  DNA
<213>  Artificial

<220>
<223>  primer

<400>  7
gagggaggcg gtaccggtgg tggctctgg                                   29


<210>  8
<211>  29
<212>  DNA
<213>  Artificial

<220>
<223>  primer

<400>  8
ccagagccac caccggtacc gcctccctc                                   29


<210>  9
<211>  25
<212>  DNA
<213>  Artificial

<220>
<223>  primer

<400>  9
gcatgctttg ccaagggtac caatg                                       25


<210>  10
<211>  52
<212>  DNA
<213>  Artificial

<220>
<223>  primer

<400>  10
ctcgagtgtg ccgttgccgt tgtttctgtc attctcataa agaattggag cg         52


<210>  11
<211>  53
<212>  DNA
<213>  Artificial

<220>
<223>  primer

<400>  11
ctcgagagaa acaacggcaa cgggaacggc acaggagatg ttttgcttaa cgt          53


<210>  12
<211>  45
<212>  DNA
<213>  Artificial

<220>
<223>  primer

<400>  12
accggtaccg cctccctcgc aattgtggac gacaacctgg gatcc                   45


<210>  13
<211>  43
<212>  DNA
<213>  Artificial

<220>
<223>  primer

<400>  13
ataagaatgc ggccgcatag agaaacaacg gcaacgggaa cgg                     43


<210>  14
<211>  20
<212>  DNA
<213>  Artificial

<220>
<223>  primer

<400>  14
taatacgact cactataggg                                               20


<210>  15
<211>  31
<212>  DNA
<213>  Artificial

<220>
<223>  primer

<400>  15
ccatggctga aactgttgaa agttgtttag c                                  31


<210>  16
<211>  27
<212>  DNA
<213>  Artificial

<220>
<223>  primer

<400>  16
ctcgaggcat gcgccaccct cagaacc                                       27


<210>  17
<211>  31
<212>  DNA

<213>  Artificial

<220>
<223>  primer

<400>  17
ccatggctga aactgttgaa agttgtttag c                                    31


<210>  18
<211>  52
<212>  DNA
<213>  Artificial

<220>
<223>  primer

<400>  18
ctcgagtgtg ccgttgccgt tgtttctgtc attctcataa agaattggag cg             52


<210>  19
<211>  40
<212>  DNA
<213>  Artificial

<220>
<223>  primer

<400>  19
tatagtatga gctcgccatg gctgaaactg ttgaaagttg                           40


<210>  20
<211>  42
<212>  DNA
<213>  Artificial

<220>
<223>  primer

<400>  20
tatatagaat tctcacttct tctcgagtgt gccgttcccg tt                        42


<210>  21
<211>  27
<212>  DNA
<213>  Artificial

<220>
<223>  primer

<400>  21
ctcgagcggc ggcggacagt ggacgcg                                         27


<210>  22
<211>  32
<212>  DNA
<213>  Artificial

<220>
<223>  primer

<400>  22
gcggccgctt accgggccag ggcctgggga gc                                   32

<210>  23
<211>  25
<212>  DNA
<213>  Artificial

<220>
<223>  primer

<400>  23
gcagccccat aaacacacag tatgt                                          25


<210>  24
<211>  92
<212>  DNA
<213>  Artificial

<220>
<223>  primer

<400>  24
atatatatgc ggccgcctta tcgtcatcgt cgtacagatc tgaaccgcct ccaccactcc    60

gctcgaggag atggattgta gcgcttatca tc                                  92


<210>  25
<211>  37
<212>  DNA
<213>  Artificial

<220>
<223>  primer

<400>  25
tataggtcga catggcttac ccatacgatg ttccaga                             37


<210>  26
<211>  36
<212>  DNA
<213>  Artificial

<220>
<223>  primer

<400>  26
tatatatcta gatagcacct tgcagcagtt gatgca                              36


<210>  27
<211>  77
<212>  DNA
<213>  Artificial

<220>
<223>  primer

<400>  27
atatatatag ccatggcggg gggttctcac caccatcacc accacgggag atctggatcc    60

gagcccaaat cttgtga                                                   77


<210>  28
<211>  45
<212>  DNA

<213>  Artificial

<220>
<223>  primer

<400>  28
gctagtcagt gcggccgcga attctttacc cggagacagg gagag                    45


<210>  29
<211>  25
<212>  DNA
<213>  Artificial

<220>
<223>  primer

<400>  29
gcagccccat aaacacacag tatgt                                          25


<210>  30
<211>  40
<212>  DNA
<213>  Artificial

<220>
<223>  primer

<400>  30
atatatatat gcggccgcga attcgaagat ccgccgccac                          40


<210>  31
<211>  34
<212>  DNA
<213>  Artificial

<220>
<223>  primer

<400>  31
tatatactcg aggaagaaaa catccagggc ggag                                34


<210>  32
<211>  32
<212>  DNA
<213>  Artificial

<220>
<223>  primer

<400>  32
tatatatcta gaaggtccat agagttcacc tg                                  32